# EUROPÄISCHE PATENTSCHRIFT

(11) **EP 3 682 913 B1**
(45) Veröffentlichungstag und Bekanntmachung des Hinweises auf die Patenterteilung: **15.12.2021**
(21) Anmeldenummer: 20152085.5
(22) Anmeldetag: 16.01.2020
(51) Int. Cl.: A61L 27/54, A61L 31/16, A61L 27/30, A61L 31/08

(54) **BAKTERIENRESISTENTE UND -ABWEISENDE, BIOKOMPATIBLE OBERFLÄCHE FÜR IN HART- UND WEICHGEWEBE INTEGRIERTE IMPLANTATE, SCHRAUBEN UND PLATTEN**
BACTERIA-RESISTANT AND REPELLENT BIOCOMPATIBLE SURFACE FOR IMPLANTS, SCREWS AND PLATES INTEGRATED INTO HARD AND SOFT TISSUE
SURFACE BIO-COMPATIBLE, RÉSISTANTE AUX BACTÉRIES ET ANTIBACTÉRIENNE POUR IMPLANTS, VIS ET PLAQUES INTÉGRÉS DANS UN TISSU DUR ET SOUPLE

(30) Priorität: 21.01.2019 DE 102019101433; 14.10.2019 DE 102019127602
(43) Veröffentlichungstag der Anmeldung: 22.07.2020
(73) Patentinhaber: Masur, Ralf, 86825 Bad Wörishofen (DE)
(72) Erfinder: Masur, Ralf, 86825 Bad Wörishofen (DE); Repenning, Detlev, 21465 Reinbeck (DE)
(74) Vertreter: Kruspig, Volkmar

(56) Entgegenhaltungen:
- DATABASE WPI Week 201757 Thomson Scientific, London, GB; AN 2017-38110E XP002799302, & CN 106 756 898 A (UNIV HUBEI) 31. Mai 2017 (2017-05-31)

## Beschreibung

Die Erfindung bezieht sich auf eine bakterienresistente und -abweisende, biokompatible Oberfläche für in Hart- und Weichgewebe integrierte Implantate, Schrauben und Platten sowie auf ein Implantat mit einer derartigen Oberfläche.

Die Patentanmeldung CN 106 756 898 A offenbart ein Verfahren zur Herstellung von antibakteriellen, hydrophoben ZnO-Nanodrähten, die auf der Oberfläche von Biomaterialien aufgebracht werden.

Bei herkömmlichen Implantatmaterialien besteht das Risiko, dass sich an ihren Oberflächen pathogene Bakterien ansammeln können, die schwere entzündliche Reaktionen hervorrufen, mit der weiteren Folge, dass sich die bakterielle Infektion im gesamten Organismus mit schwerwiegenden Folgen ausbreiten kann. Besonders gravierend ist die mangelnde Resistenz mit dem Einsatz herkömmlicher Implantat Materialien bei immunsupressiven Patienten. Vielfach ist es nicht möglich, diesen Patienten Implantate oder Fixationssysteme bei Knochenbrüchen u.ä. zu implantieren.

Als Lösung werden gegenwärtig Oberflächen mit Silber- oder Kupferdotierungen in den Oberflächen angeboten. Diese werden beispielsweise in Keramiken, Hydrolylapaptit-Oberflächen u.a.m. eingebracht. Die Nanometer großen stark antibakteriell wirkenden Partikel stehen jedoch im Verdacht auch zytotoxisch zu wirken oder auch die Osseointegration von Implantaten zu verhindern bzw. deutlich zu verlangsamen.

Weiterhin ist aus Untersuchungen bekannt, dass beispielsweise typische Titan GrII oder GrIII - Materialien, die im Bereich der Dentalimplantate genutzt werden, von pathogenen Bakterien korrosiv angegriffen werden.

Der langfristige Erfolg eines Zahnimplantates hängt im Wesentlichen von der nano- und mikrogeometrischen Auslegung der Oberflächen und den makrogeometrischen Gestaltungen des Zahnimplantats im zervikalen und im transgingivalen Bereich des Implantates ab.

Besondere Bedeutung für den Langzeiterfolg eines Implantats ist der Auswirkung der Plaque-Bildung an der Implantatoberfläche auf die periimplantäre Mukosa zuzumessen. Vielfach wird insbesondere im Zusammenhang mit einer schlechten Mundhygiene bei Titan-Implantaten eine periimplantäre Mukositis beobachtet, also eine reversible Entzündung um das funktionell belastete Implantat. Eine lang andauernde Plaque-Bildung ruft ein Infiltrat von Entzündungszellen in der Mukosa hervor, die zur weiteren Ausbreitung der mikrobiellen Taschenflora führt (Ericsson, 1995, 1996). Aufgrund ihrer schlechten Durchblutung weist die periimplantäre Mukosa einen verminderten Abwehrmechanismus auf im Vergleich zur Gingiva. Aus dem Voranschreiten der Weichgewebsläsion bis zum Implantat und ins Knochenmark resultiert der vorzeitige Implantatverlust. Entzündliche Reaktionen der beschriebenen Art stellen die häufigste Verlustursache von Implantaten nach dem Einheilungsprozess dar (Koutsonikos, 1998; Esposito, 1999; Berglundh et al., 2002).

Die Anforderungen an Oberflächen von Zahnimplantaten müssen wegen der im Kiefer und in der Mundhöhle unterschiedlichen Bedingungen getrennt voneinander formuliert werden. Für das in den Kiefer eingebrachte Teil ist eine Oberfläche gefordert, die mindestens der Biokompatibilität und Osteophilität der passivierenden Oxidschicht auf Titan entspricht (Kasemo/Lausmaa, 1985; Bowers et al., 1992, Cochran, 1994). Allerdings soll die Entzündungsempfindlichkeit gegenüber Titan/Titandioxid gemindert werden.

In Bezug auf die Plaque-Bildung an Implantat-Oberflächen werden Versuche unternommen, "Antiplaque-Mechanismen" zu definieren. Thull (1991, 1992) erklärt elektrisch leitende Oberflächen als geeignet zur Vermeidung der Plaqueanlagerung. Hohe elektrische Feldstärken am Interface "anorganische Oberfläche - biologisches Milieu" sollen die Denaturierung adsorbierter Makromoleküle bewirken. Krämer et al. (1989) kommen zum gegenteiligen Schluss, nämlich dass mit dicken biologischen Titanoxidschichten eine geringere Plaque-Akkumulation stattfindet als mit solchen Oberflächen, die zur Denaturierung des Proteins führen.

Insgesamt erscheinen solche Definitionen mit Erkenntnis der Bakterienanlagerung an Oberflächen sehr vereinfacht und genügen nicht den komplexen, unsymmetrischen Mechanismen der bakteriellen Adhäsion. Implantatoberflächen stellen einen der beiden wechselwirkenden Partner dar im Prozess der primären Bakterienadhäsion. Dabei gelten für das Trägermaterial die gleichen physikochemischen Gesetzmäßigkeiten wie für die generell zu beschreibenden Mechanismen mit allen Oberflächen in biologischen und nicht-biologischen Oberflächen. Es spielen wie bei den bakteriellen Oberflächen die Hydrophobizität oder -philie und die elektrostatischen Eigenschaften eine wichtige Rolle.

Die Mehrzahl der Forschungen und praktischen Erfahrungen zielen darauf ab, wie günstige Oberflächen zur Anheftung von Bakterien beispielsweise in Kläranlagen geschaffen werden können. In der Implantologie mit den Fremdkörpersituationen sind gleichsam inverse Forderungen verbunden, die besonders darauf abzielen, die Adhäsion pathogener Keime zu verhindern oder u.U. sogar zu denaturieren. Gleichzeitig gilt die Maßgabe, die für das Knochenwachstum oder die Adhäsion der Gingiva förderlichen Zellen zu adhärieren und Voraussetzungen für deren Proliferation zu schaffen.

Den allgemein zugrundeliegenden Mechanismus bei der supragingivalen Plaquebildung beschreiben Quirynen/Bollen (1995) mit vier verschiedenen Phasen als Basis für die grundlegende Anhaftung von Bakterien an Fremdoberflächen:
1. Transport der Bakterien zur Oberfläche
2. Initiale Adhäsion mit einer reversiblen und irreversiblen Phase
3. Anheftung an die Oberfläche
4. Kolonisation mit Bildung eines Biofilms

Der Transport der Bakterien zu der zu besiedelnden Oberfläche kann auf drei verschiedene Arten erfolgen: Mittels Brownscher Bewegung, konvektiven Transports durch den Flüssigkeitsstrom und chemotaktischer aktiver Bakterienbewegung.

Die initiale Adhäsion der Bakterien an die verschiedenen Oberflächen erfolgt durch relativ unspezifische, physikochemische Wechselwirkungen. Hierbei wirken unter anderem elektrostatische Kräfte mit hydrophoben Wechselwirkungen.

Dieser erste Schritt der initialen Anheftung ist reversibel. Danach bildet sich eine feste, irreversible Verbindung zwischen dem Bakterium und der Oberfläche, die durch spezifische Interaktionen wie z. B. kovalente Bindung, Ionen- oder Wasserstoffbindung den direkten Kontakt mittels spezieller extrazellulärer bis zu 10 nm langen Haftorganellen (Filamentartige Fortsätze, Fimbrien oder Pili) hergestellt wird. Die extrazellulären Proteinkomponenten der Organismenzellwand, die eine Bindung mit der Oberfläche eingehen, werden auch als Adhäsine bezeichnet.

Das ca. acht Mikrometer dicke Pellikel oder Zahnoberhäutchen besteht vor allem aus Glucoproteinen des Speichels und bildet die Matrix für die ersten siedelnden Bakterien. Dabei erfolgt die Anlagerung der Bakterien höchst selektiv. Beim Prozess der Plaquereifung wird über Rezeptoren der Pionierkeime wie Streptokokkus, Sanguis und Mutans eine weitere Anlagerung von Keimen möglich. Auf jeder gereiften Schicht bildet sich wiederum ein Pellikel, so dass es zu einem Schichtaufbau der reifen Plaque mit ihrer typischen Bakterienzusammensetzung kommt:
Um den oben beschriebenen Mechanismus besser zu verstehen, sind im Folgendem die physiko-chemischen Schritte zusammengefasst:

### A. Die Bedeutung der extrazellulären und oberflächengebundenen Polysaccharide

1. Die elektrostatischen Wechselwirkungen ergeben sich aus den vorwiegend negativ geladenen Oberflächen der Bakterien, die zu großen Anteilen aus Sacchariden mit negativ geladenen Carboxylgruppen besteht.
2. Die hydrophobe Wechselwirkung findet auch an hydrophilen Oberflächen statt und begründet sich thermodynamisch mit der Absenkung der freien Energie und einem entsprechenden Entropiegewinn.
3.1 Für den Prozess der primären Adsorption besitzen oberflächengebundene Polysaccharide eine herausragende Rolle. Nach erfolgter initialer Anheftung setzt üblicherweise eine intensive Synthese extrazellulärer Substanzen und vor allem von Polysacchariden ein, die zur irreversiblen Anheftung der Zellen führt und zur Ausbildung des reifen Biofilms.
3.2 Verschiedene Untersuchungen weisen darauf hin, dass auch oberflächengebundene Polysaccharide mit an der initialen Adhäsion beteiligt sind.
4.1 Besonders unter anaeroben oder semianaeroben Bedingungen wirken die oberflächengebundenen Polysaccharide mit, welche offensichtlich die Adsorption der Organismen an inerten Oberflächen fördert.
4.2 bei den Gram+ Bakterien, zu denen besonders die pathogenen Bakterienarten Streptokokken und Staphylokokken gehören, sind zu großen Anteilen die stark hydrophile Teichon- und Teichuronsäuren in der Oberfläche der Organismen vertreten. Phosphate, Hydroxyl-, und Carboxylgruppen sind die makroskopisch wirksamen Gruppen in den langkettigen Molekülen. Sie sind anionisch und sauer. In den Seitenketten sind Aminosäuren gebunden, die die kovalente Bindung mit einer Wirtsunterlage ermöglichen.
5. Summarisch kann aufgrund der asymmetrisch angeordneten Oberflächenpolysaccharide eine Vielzahl von Bakterien sowohl an hydrophoben als auch an hydrophilen Oberflächen adsorbieren. Die Anhaftung an hydrophobe Oberflächen findet dabei überwiegend an den polysaccharidfreien Zonen der Organismenoberfläche statt.

### B. Die Bedeutung der Proteine

Neben den Polysacchariden gehören die Proteine zu den wichtigsten Bestandteilen der Bakterienoberfläche. Sie sind als Membranproteine sowie Grundbausteine von Fibrien und Flagellen direkt am Zellaufbau beteiligt. Mit über 40% stellen sie einen wichtigen Bestandteil der Extrazellulären Polysaccharide dar. Ihre hydrophoben Seitenketten beeinflussen die oben beschriebenen hydrophoben Eigenschaften der Organismen erheblich.

Die proteinogenen Adhäsine der EPS (ExtrazellulärePolySaccharide) - wie z.B. an Zahnoberflächen- sind nicht nur an der Adhäsion beteiligt, sondern sie können auch enzymatische Aktivität entwickeln und auf diese Weise zur Schaffung einer optimalen Struktur der Wirtsoberfläche beitragen.

Zusammenfassend ergeben sich für die Anhaftung von Bakterien an Oberflächen folgende Kriterien, die zur Auswahl des geeigneten Implantatwerkstoffes zu beachten sind:
1. Bakterien weisen aufgrund des hohen Anteil von Polysacchariden mit Carboxyl-, Phosphat- und Hydroxylgruppen eine stark negativ geladene Oberfläche auf.
2. Nach primärer Adsorption bilden sie einen extrazellulären Polysaccharidfilm aus, der sich an der Oberfläche anlagert und die Wirtsmatrix für das weitere Wachstum der Kolonisation sorgt. Hiermit wird die Oberfläche konditioniert ("conditioning film").
3. Im sauerstoffarmen Habitat nutzen Bakterien ebenso das eigene Oberflächen gebundenen Polysaccharid zur Adhäsion.
4. Der hohe Proteinanteil in den Sacchariden ermöglicht auch die hydrophobe Anhaftung der Bakterien.
5. Die proteinogenen Adhäsine können enzymatische Aktivität entwickeln und somit bestmögliche Strukturen auf der Wirtsoberfläche bilden.
6. Gram negative Bakterien besitzen an der Zelloberfläche als strukturbildenen Bestandteil Lipopolysaccharide. Untersuchungen weisen darauf hin, dass die Struktur der Lipopolysaccharide eng mit der Hydrophobizität der Organismen geprägt ist. Ihnen werden zudem komplexbildende Eigenschaften zugeschrieben.
7. Bakterien und insbesondere die pathogenen Spezies weisen Größen von 0,6 bis 1,2 Mikrometer auf. Sie sind entweder als Kugeln zu langen perlenähnlichen Ketten verbunden (Staphylokokkus) oder die kugeligen Einzelorganismen bilden Kugelhaufen. Sie sind gram+.

Aus den genannten Kriterien ist zu schließen, dass im Prinzip an nahezu allen Oberflächen eine Anlagerung der Bakterien zu erwarten ist. Eine besondere Rolle spielen hierbei die Kunststoffe, da sie nicht nur physikalisch-chemisch und konstitutionell die Anlagerung fördern, sondern sie lassen sich auch durch die enzymatische Aktivität der Bakterien leicht strukturell und konstitutionell umbauen. Selbst bei anorganischen Oxidoberflächen wie bei den Titanoxiden zeigen Untersuchungen, dass diese durch die sauer wirkenden anaerob wirkenden Bakterien "angegriffen" werden und zu voluminösen schwarzen Suboxiden reduziert werden.

Angesichts der oben beschriebenen Nachteile der aus dem Stand der Technik bekannten Lösungen stellt sich die vorliegende Erfindung die Aufgabe, Implantatoberflächen derart weiterzubilden, dass die oben beschriebenen Nachteile reduziert oder vermieden werden.

Die erfindungsgemäße Aufgabe wird durch eine Oberfläche für Implantatoberflächen gemäß den Merkmalen von Anspruch 1 gelöst. Vorteilhafte Weiterbildungen ergeben sich aus den Unteransprüchen.

Mit der vorliegenden Erfindung werden entsprechend mehrere Effekte erzielt:
- An den ihr zugrundeliegenden Oberflächen haften sich insbesondere keine pathogenen, anaeroben oder "bivalenten" Bakterien an.
- Die Oberflächen vermögen besonders die pathogenen Bakterien zu denaturieren ohne dabei die Zellanlagerung zur Adhäsion des Weich- oder Hartgewebes zu beeinträchtigen.
- Die beschriebenen Oberflächen sind biochemisch bzw. chemisch resistent gegen die stark saurer wirkenden Oberflächenmoleküle der pathogenen Bakterien sowie ihre enzymatischen Umwandlungseigenschaften.
- Die Oberflächen wirken nicht zytotoxisch wie etwa die "antibakteriell" wirkenden Metalle Kupfer oder Silber.
- Die Oberflächen sind strukturiert negativ oder vollständig negativ geladen und wirken somit repulsiv auf die Bakterien.
- Die Oberflächen sind mehrphasig und sind in definierten Nanometer- und Mikrometergroßen Bereichen unterschiedlich elektrisch geladen und wirken somit lateral polarisierend auf das Bakterium.

### Herstellung der Oberflächen und ihre thermodynamisch bestimmte chemische Stabilität

Grundlage zur Auswahl der Oberflächen sind die chemisch hochstabilen Oxide der Metalle aus der IV. und V. Nebengruppe des Periodischen Systems der Elemente (PSE). In der nachstehenden Tabelle 1 sind die freien Reaktionsenthalpien der oxidbildenen Elemente dargestellt. Diese zeigen, dass besonders Tantaloxid und Nioboxid sehr stabile Oxide bilden. Die freien Bildungsenenthalpien von Titandioxid liegen deutlich niedriger.

| **Freie Bildungsenthalpien verschiedener Refraktärmetalloxide** | |
|---|---|
| **Verbindung** | **ΔG (kJ/mol)** |
| **Titandioxid** | **-956** |
| **Zirkondioxid** | **-1101** |
| **Niobpentoxid** | **-1951** |
| **Tantalpentoxid** | **-2064** |
| **Tabelle 1** | |

Ein nicht erwünschtes Kriterium ist die Möglichkeit der Oxide zum Valenzwechsel der Metalle in den Verbindungen. Besonders für Titan sind je nach Milieubedingungen Suboxide bekannt, bis hin zu den elektrisch leitenden "Magneliphasen". Titan kann dabei positive Wertigkeiten zwischen 2 und 4 annehmen. Selbst bei dem vierwertigen Titan im Titandioxid bilden sich schnell bei leicht reduktiver Bedingungen unterstöchiometrische Verbindungen des Typus TiO₂₋ₓ. Die schwarz gefärbten Suboxide sind evident und werden bei herkömmlichen Oberflächen vielfach im klinischen Alltag beobachtet. Sie treten vermehrt an der Verbindung zwischen Abutement und enossalem Implantat auf.

Weitere Maßgabe zur Auswahl der Oberfläche stellen die pH-abhängigen pzzp's der Oxide *(point of zero zeta potential)* dar: Oxide binden Wasser an der Oberfläche, um die Sauerstoff-Koordination an der Oberfläche zu vervollständigen. Die Protonen des "gebundenen" Wassers sind verteilt über die äquivalenten Sauerstoffatome und stehen im Gleichgewicht mit dem pH des jeweiligen wässrigen Mediums. Bei pH-spezifischen Werten der jeweiligen Oxide sind die Oberflächen neutral geladen. Der pH am neutralen Punkt wird pzzp (point of zero zeta potential) genannt. Bei pH<pzzp binden die Oberfläche einen Überschuss an Protonen und sind entsprechend positiv geladen, bei pH>pzzp geben die Oberflächen Protonen ab und sind entsprechend negativ geladen.

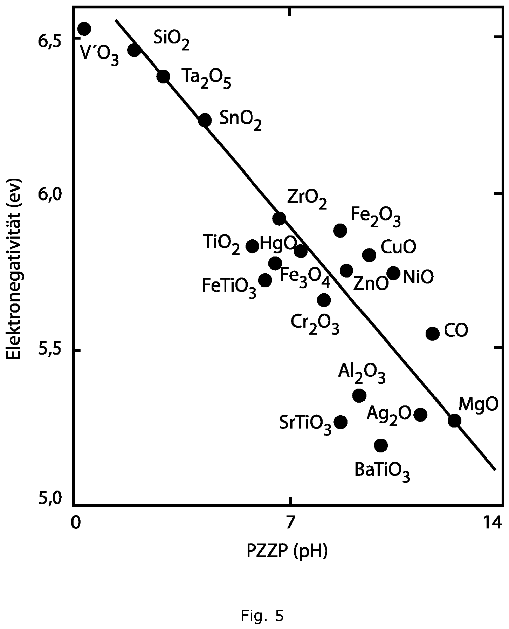

Die pzzp's der infrage kommenden Oxide sind in Figur 5 dargestellt. Der pH für die elektrische Neutralität von Tantaloxid liegt bei 1. Somit sind Tantaloxide oberhalb dieses pH-Wertes negativ geladen. Das Oxid wirkt entsprechend repulsiv auf die Bakterien. Pro pH bildet sich nach Maßgabe der Nernst Relation ein Oberflächenpotential von ca. 60mV (bezogen auf 36 Grad Celsius physiologisch) aus. Hieraus ergibt sich beispielsweise für Tantaloxid unter physiologischen Bedingungen bei pH 7,2 ein Oberflächenpotential von -372mV.

Der pzzp von Titanoxid in der Anatas Modifikation liegt bei pH 6,4. Zirkonoxid hat einen pzzp von 5 und bei Nioboxid liegt er bei pH 3 bis pH 4. Hafniumoxid weist den Neutralpunkt im alkalischen bei pH 8,4 auf ebenso wie beispielweise Aluminiumoxid einen Wert im alkalischen aufweist.

Eine Feinjustierung des pzzp's erfolgt durch Mischphasenbildung der ausgewählten Oxide.

Die Mischphasenbildung wird erfindungsgemäß in einer weiteren möglichen Ausführungsform zur Bildung einer zweiphasigen Oberfläche genutzt und damit zur Einstellung der mindestens bipolaren Oberfläche. Hieraus entsteht an der Oberfläche ein definiertes elektrisches Muster mit einstellbaren elektrischen Feldstärken zwischen den einzelnen Phasen. Durch die Einstellung beispielsweise eines ternären Mischphasensystems ist es möglich ein komplexes elektrisches "oberflächenfluides", wiederholbares Muster zu erzeugen. Die Oberflächenladungen und an den Korngrenzen erzeugten elektrischen Gradienten fungieren als Leitstrukturen und üben einen positiven Reiz für Osteoblasten aus.

Anders als bei Bakterien fördern negativ geladene Oberflächen Zelladhäsion und Zellfunktion wesentlich stärker als positiv geladene Oberflächen.

Mit der variablen Einstellung der einzelnen Phasen in Korngrößen in Nanometerbereich zwischen 1 und 100 nm wird die biochemische Aktivität für das Einwachsen des Implantats gezielt beeinflusst. Die Benetzbarkeit sowie die spezifische Adsorption von Proteinen und möglichen Mineralisationsmechanismen werden günstig beeinflusst. Die Mechanismen bewirken veränderte Zelladhäsionen an der Oberfläche, indem sowohl Integrin und Nicht-Integrin Rezeptoren für die Zellbindung miteingeschlossen werden.

Ein wesentlicher Aspekt einiger Ausführungsformen der erfindungsgemäßen Ausführung der Oberfläche ist, dass sie einerseits eine chemisch hohe Stabilität aufweisen und bei einer eindringenden bakteriellen Besiedlung von den Bakterien nicht konditioniert wird und andererseits, dass die Oberfläche in nanoskalierter Dimension positive Reize für die Zelladhäsion und deren Proliferation ausübt. Die hohe chemische Stabilität ist eine *conditio sine qua non.*

Nachfolgend wird die Erfindung auch hinsichtlich weiterer Merkmale und Vorteile anhand von Ausführungsbeispielen beschrieben, die anhand der Figuren näher erläutert werden.

Hierbei zeigen:
- Fig. 1: eine schematische Darstellung der oberflächlichen Ladungsmuster und -strukturen an einer bipolaren Oberfläche;
- Fig. 2: ein Phasendiagramm des Systems ZrO₂-TiO₂;
- Fig. 3: ein Phasendiagramm des Systems Niobpentoxid -Tantalpentoxid;
- Fig. 4: ein Phasendiagramm des Systems Tantalpentoxid - Titandioxid; und
- Fig. 5: die pzzp's verschiedener Oxide.

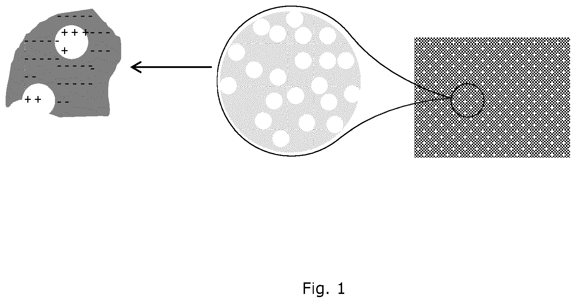

Figur 1 zeigt eine schematische Darstellung der oberflächlichen Ladungsmuster und -strukturen an einer bipolaren Oberfläche. Die graue Oberfläche stellt eine Matrix mit hoher Dichte an negativer Ladung, wie beispielsweise eine Tantaloxid-Matrix, dar. In weiß sind eingelagerte, im Zweiphasengebiet ausgeschiedene, Körner mit positiver Oberflächenladung, wie beispielsweise (Ti₉₅Ta₅)O₂ in einem Medium bei pH 5 dargestellt. Die Größe der Körner beträgt beispielsweise etwa 80 nm.

### a) Herstellung einer zweiphasigen Titan-Zirkonoberfläche

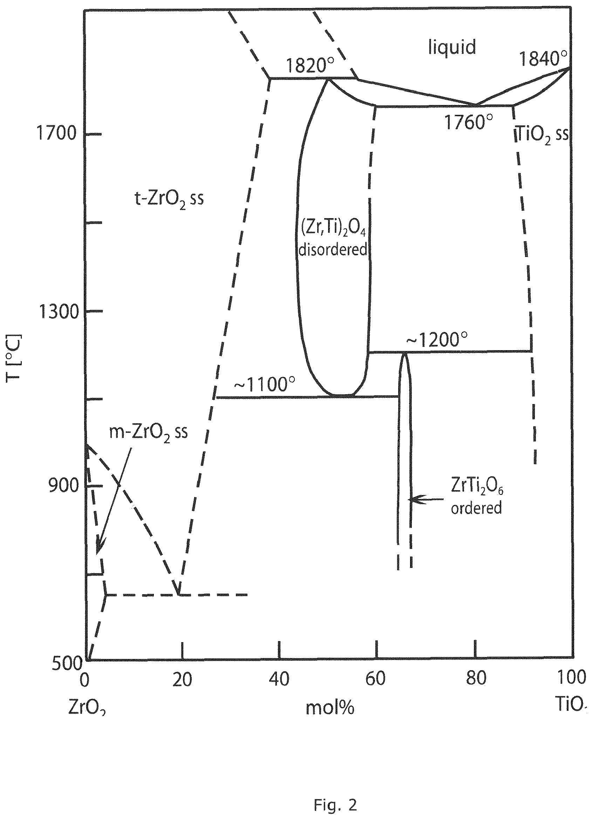

In Figur 2 ist das Phasendiagramm des Systems ZrO₂-TiO₂ dargestellt. Es ist die Temperatur in °C gegen das Stoffmengenverhältnis der Bestandteile ZrO₂ und TiO₂ zueinander dargestellt, wobei auf der Abszisse der prozentuale Stoffmengenanteil von Titandioxid in mol% aufgetragen ist. Das Phasendiagramm verdeutlicht, dass bei physiologischer Temperatur Zirkondioxid eine zu vernachlässigende Löslichkeit für Titandioxid aufweist, das gleiche gilt für Titandioxid in Bezug auf Zirkondioxid. Das System weist hingegen eine geordnete Phase ZrTi₂O₆ auf. Die freie Bildungsenergie liegt temperaturabhängig bei ca. 1000J/mol. Hiermit ist eine deutliche Stabilisierung des 4-wertigen Titans in der Mischphase erreicht. Für die erfindungsgemäße Beschichtung wurde eine mittlere Zusammensetzung mit 40mol% TiO₂ gewählt.

Nach Maßgabe des Hebelgesetzes bilden sich bei Tempern der Schicht bei 370 K 38mol% der ZrO₂-Phase und 62mol% der ZrTi2O₆-Phase aus. Die Korngrößen der einzelnen Phasen wurden mit durchschnittlich 42 nm bzw. 65 nm bestimmt. Biokompatibilitätsuntersuchungen können beispielsweise nach dem ELISA-Verfahren (Prof. R. Thull, "Biologische und tierexperimentelle Prüfung von (Ti,Zr)O2 und (Ti,Nb)ON Schichtwerkstoffen auf Titan"; Lehrstuhl für experimentelle Zahnmedizin Juni 1992) bestimmt werden. Die hier beschriebenen Oberflächen weisen Stabilitätsindices von 1 aus. Mittels Kelvinsonde wurden an der Oberfläche Potentiale von -126 mV und -18mV für die ternäre Phase ermittelt.

### b) Herstellung einer zweiphasigen Ta₂O₅-Nb₂O₅-Oberfläche

Die Anwendung von Tantaloxid-haltigen Oberflächen ist aus mehreren Gründen von besonderem Interesse:
- Tantaloxid gilt als besonders biokompatibel bis hin als bioaktiv.
- Tantaloxid weist den pzzp bei pH 1 aus, das Oxid ist also unter physiologischen Bedingungen hoch negativ aufgeladen, mit einem Oberflächenpotential von ca. -370mV.
- Tantaloxid ist das stabilste Oxid unter allen natürlich vorkommenden Oxiden.
- Nioboxid ist ähnlich stabil wie Tantaloxid und biokompatibel.

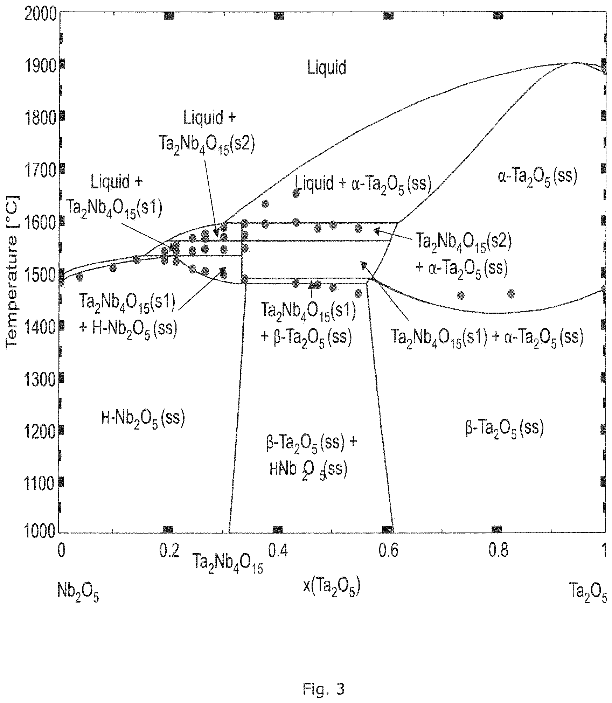

Das Phasendiagramm in Figur 3 zeigt, dass im System NbzOs-TazOs eine Mischungslücke zwischen ca. 32 Mol% und 62Mol% Tantaloxid auftritt. Die Schichten wurden zunächst auf der tantalreichen Seite im Phasendiagramm eingestellt, mit einer Variation des Niobgehalts zwischen 0% und 50%. Letztere Einstellung diente der Herstellung der bipolaren Oberfläche. Einstellungen unterhalb von 38 Mol% sind einphasig und sollten den Einfluss des Niobgehalts auf den pzzp untersuchen. Die pzzp's variieren in der festen Lösung beta-Tantaloxid-ss zwischen pH 1 und pH 1,5, auf der H-Nioboxidseite stellt sich an der Phasengrenze ein pzzp von ca. pH 2,5 ein.

### c.) Herstellung einer zweiphasiaen TiO₂-Ta₂O₅-Oberfläche

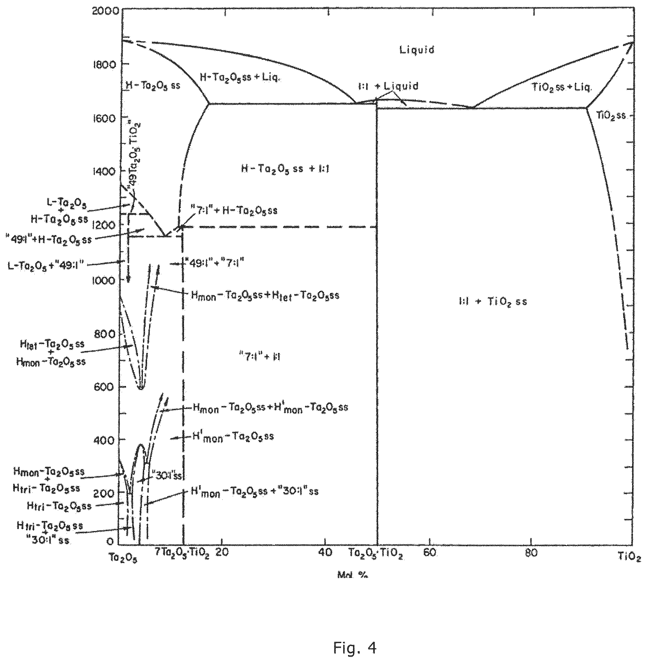

Das Phasendiagramm in Figur 4 weist eine Löslichkeit von TiO₂ in Ta₂O₅ von ca. 12 Mol% aus und eine feste Verbindung TiO₂-Ta₂O₅ entsprechend je 50 Mol% aus den beiden Oxiden. Als Schicht wurde u.a. eine Zusammensetzung mit 40 Mol% TiO₂ gewählt, entsprechend einer Zusammensetzung 73% TiO₂-Ta₂O₅ und 27% Ta₂O₅-ss mit 12% TiO₂. Die pzzp's der einzelnen Phasen wurden mit pH 4-5 und pH 1-2 bestimmt. Die Korngrößen wurden auf 45 nm und 90 nm eingestellt. Die makroskopische Rauigkeit an der Implantatoberfläche wurde mit Rₐ 2-3 Mikrometer gewählt.

Nach Maßgabe der Molanteile der einzelnen Phasen ergibt sich hieraus ein Kornverhältnis von 6:1 zugunsten der negativ geladenen Ta₂O₅-ss-Phase. Die 90 nm großen TiOz-TazOs-Körner sind demgemäß von der 6-fachen Menge der kleineren Ta₂O₅-ss Körner umgeben. An den Grenzflächen dieser Körner bildet sich ein elektrischer Feldgradient aus, der sich aus der Potentialdifferenz von 180mV ergibt. Zudem bildet sich an der TiO₂-Ta₂O₅ eine höhere Hydrophilie aus als an der tantalreichen Phase.

Der Biokompatibilitätsindex dieser Oberfläche wurde mittels ELISA-Verfahren mit nahe 1 bestimmt und die materialinduzierte Entzündlichkeit lag bei 5. Anders als auf einer Titanoberfläche Gr4 werden bei einer 7-tägigen intraoralen Exposition mittels einer Miniplastschiene mit insgesamt 156 Probekörpern bei 13 Probanden als Trägervorrichtung auf dieser Schicht keine Bakterienagglomerate beobachtet. Die Titanoberflächen sind hingegen mit einem dichten Film aus Mikroorganismen besiedelt. Nach etwa einer Woche finden sich auch Spirochäten und bewegliche Stäbchen an der Titanoberfläche, hier hat sich die reife Plaque ausgebildet.

## Patentansprüche

1. Bakterienresistente und -abweisende, biokompatible, chemisch stabile Oberfläche für Implantatoberflächen,
**dadurch gekennzeichnet, dass** sie mehrphasig ist bzw. eine heterogene Mischphase ausbildet, und mindestens bipolar ist, wobei mindestens eine Phase einen pzzp in physiologischer Umgebung von 5 oder mehr aufweist, wobei die Oberfläche als Mischoxide der Elemente der IV. und V. Nebengruppe des Periodensystems ausgebildet ist.

2. Oberfläche gemäß Anspruch 1, wobei die Mischoxide mindestens zweiphasig ausgebildet sind.

3. Oberfläche gemäß einem der vorhergehenden Ansprüche, wobei die Mischoxide in den Phasen binär und in höherer Koordination vorliegen.

4. Oberfläche gemäß einem der vorhergehenden Ansprüche, wobei die Mischoxide auf den Anionenplätzen zusätzlich die Elemente Fluor und/oder Stickstoff und/oder Kohlenstoff und/oder Bor und/oder Phosphor enthalten.

5. Oberfläche gemäß einem der vorhergehenden Ansprüche, wobei die Konzentrationen der Nichtmetallelemente zwischen x=0 und x=1 liegen, bezogen auf die Sauerstoffkonzentration gemäß der vereinfachten und stellvertretenden Formulierung MeO₂₋ₓYₓ wobei Me die Metalle der Mischoxide repräsentieren und Y die Nichtmetalle der Mischoxide auf den Anionenplätzen.

6. Oberfläche gemäß einem der vorhergehenden Ansprüche, wobei die Oberfläche zusätzlich Calcium in der Konzentration x=0 und x=0,2 bezogen auf die anderen mischoxidbildenden Metalle, enthält.

7. Oberfläche gemäß einem der vorhergehenden Ansprüche, wobei die Oxide der heterogenen Mischphase unterschiedliche pzzp's aufweisen.

8. Oberfläche gemäß einem der vorhergehenden Ansprüche, wobei die pzzp's der einzelnen Oxide pzzp-Werte zwischen pH=0 und pH=9 in physiologischer Umgebung aufweisen.

9. Oberfläche gemäß einem der vorhergehenden Ansprüche, wobei die Korngrößen der einzelnen Oxide in den heterogenen Mischphasen zwischen 1 nm und 20 Mikrometer liegen.

10. Oberfläche gemäß einem der vorhergehenden Ansprüche, wobei die Oberfläche variabel hydrophil und/oder hydrophob eingestellt ist.

11. Oberfläche gemäß einem der vorhergehenden Ansprüche, wobei die Oberflächenenergie der mehrphasigen Oberfläche zwischen s = 18mN/m und 65mN/m liegt, entsprechend einem Wasserkontaktwinkel von 25° und 120°.

12. Implantat, aufweisend eine Oberfläche gemäß einem der Ansprüche 1 bis 11.

## Claims

1. A bacteria-resistant and bacteria-repellent, biocompatible, chemically stable surface for implant surfaces,
**characterized in that** it is multiphase or forms a heterogenous mixed phase, and is at least bipolar, wherein at least one phase has a pzzp in a physiological environment of 5 or more, wherein the surface is formed as mixed oxides of the elements of the IVth and Vth subgroup of the periodic system.

2. The surface according to claim 1, wherein the mixed oxides are formed to be at least two-phase.

3. The surface according to any one of the preceding claims, wherein the mixed oxides in the phases are present in binary form and in higher coordination.

4. The surface according to any one of the preceding claims, wherein the mixed oxides contain in addition the elements fluor and/or nitrogen and/or carbon and/or boron and/or phosphorus in the anion positions.

5. The surface according to any one of the preceding claims, wherein the concentrations of the non-metallic elements are between x = 0 and x = 1 with respect to the oxygen concentration according to the simplified and representative formulation MeO₂₋ₓYₓ, wherein Me represents the metals of the mixed oxides and Y represents the non-metals in the anion positions.

6. The surface according to any one of the preceding claims, wherein the surface contains in addition calcium in the concentration x = 0 and x = 2 with respect to the other metals forming mixed oxides.

7. The surface according to any one of the preceding claims, wherein the oxides of the heterogenous mixed phase have different pzzps.

8. The surface according to any one of the preceding claims, wherein the pzzps of the individual oxides have pzzp values of between pH = 0 and pH = 9 in a physiological environment.

9. The surface according to any one of the preceding claims, wherein the grain sizes of the individual oxides in the heterogenous mixed phases are between 1 nm and 20 micrometers.

10. The surface according to any one of the preceding claims, wherein the surface is adjusted to be variably hydrophilic and/or hydrophobic.

11. The surface according to any one of the preceding claims, wherein the surface energy of the multiphase surface is between s = 18 mN/m and 65 mN/m corresponding to a water contact angle of 25° and 120°.

12. An implant comprising a surface according to any one of claims 1 to 11.

## Revendications

1. Surface biocompatible chimiquement stable résistante aux bactéries et antibactérienne pour surfaces d'implant,
**caractérisée en ce qu'**elle est multiphasée ou constitue une phase mixte hétérogène, et est au moins bipolaire, sachant qu'au moins une phase présente un pzzp en milieu physiologique de 5 ou plus, sachant que la surface est constituée comme oxydes mixtes des éléments du IVe et du Ve sous-groupe du système périodique.

2. Surface selon la revendication 1, sachant que les oxydes mixtes sont constitués au moins de manière biphasée.

3. Surface selon l'une des revendications précédentes, sachant que les oxydes mixtes sont présents dans les phases de manière binaire et en coordination supérieure.

4. Surface selon l'une des revendications précédentes, sachant que les oxydes mixtes contiennent en outre les éléments fluor et/ou azote et/ou carbone et/ou bore et/ou phosphore aux emplacements anioniques.

5. Surface selon l'une des revendications précédentes, sachant que les concentrations en les éléments non métalliques sont comprises entre x = 0 et x = 1, par rapport à la concentration en oxygène selon la formulation simplifiée et de substitution MeO₂₋ₓYₓ, sachant que Me représente les métaux des oxydes mixtes et Y les non-métaux des oxydes mixtes aux emplacements anioniques.

6. Surface selon l'une des revendications précédentes, sachant que la surface contient en outre du calcium dans la concentration x = 0 et x = 0,2, par rapport aux autres métaux qui forment des oxydes mixtes.

7. Surface selon l'une des revendications précédentes, sachant que les oxydes de la phase mixte hétérogène présentent des pzzp différents.

8. Surface selon l'une des revendications précédentes, sachant que les pzzp des divers oxydes présentent des valeurs pzzp comprises entre pH = 0 et pH = 9 en milieu physiologique.

9. Surface selon l'une des revendications précédentes, sachant que les tailles de grain des oxydes individuels dans les phases mixtes hétérogènes sont comprises entre 1 nm et 20 micromètres.

10. Surface selon l'une des revendications précédentes, sachant que la surface est ajustée pour être variablement hydrophile et/ou hydrophobe.

11. Surface selon l'une des revendications précédentes, sachant que l'énergie de surface de la surface multiphasée est comprise entre s = 18 mN/m et 65 mN/m, selon un angle de contact d'eau de 25° et 120°.

12. Implant, présentant une surface selon l'une des revendications 1 à 11.
